(19) Europäisches Patentamt
European Patent Office
Office européen des brevets

(11) **EP 3 027 173 B1**

(12) **EUROPEAN PATENT SPECIFICATION**

(45) Date of publication and mention
of the grant of the patent:
**25.07.2018 Bulletin 2018/30**

(21) Application number: **14781670.6**

(22) Date of filing: **01.08.2014**

(51) Int Cl.:
*A61K 9/00* (2006.01)        *A61K 9/70* (2006.01)
*A61K 31/522* (2006.01)      *A61P 11/06* (2006.01)
*A61P 11/08* (2006.01)

(86) International application number:
**PCT/IB2014/063610**

(87) International publication number:
**WO 2015/015459 (05.02.2015 Gazette 2015/05)**

(54) **TRANSDERMAL ADMINISTRATION SYSTEM FOR USE IN THE TREATMENT OF CHRONIC BRONCHOPULMONARY DISEASES**

TRANSDERMALES VERABREICHUNGSSYSTEM ZUR VERWENDUNG BEI DER BEHANDLUNG DER CHRONISCHEN BRONCHOPULMONALEN ERKRANKUNG

SYSTÈME D'ADMINISTRATION TRANSDERMIQUE UTILISABLE DANS LE CADRE DU TRAITEMENT D'AFFECTIONS BRONCHO-PULMONAIRES CHRONIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.08.2013 IT TO20130663**

(43) Date of publication of application:
**08.06.2016 Bulletin 2016/23**

(73) Proprietor: **ABC Farmaceutici S.p.A.**
**10121 Torino (IT)**

(72) Inventors:
• **GIRAUDI, Alberto**
  **I-10090 Cinzano (Torino) (IT)**
• **GIRAUDI, Giovanni**
  **I-10129 Torino (IT)**

(74) Representative: **Rambelli, Paolo et al**
**Jacobacci & Partners S.p.A.**
**Corso Emilia 8**
**10152 Torino (IT)**

(56) References cited:
**WO-A2-2007/066151      WO-A2-2007/120485**

• THAKUR RASHMI A ET AL: "Transdermal and buccal delivery of methylxanthines through human tissue in vitro.", DRUG DEVELOPMENT AND INDUSTRIAL PHARMACY MAY 2007, vol. 33, no. 5, May 2007 (2007-05), pages 513-521, XP002722924, ISSN: 0363-9045 cited in the application
• ADEMOLA J I ET AL: "CUTANEOUS METABOLISM OF THEOPHYLLINE BY THE HUMAN SKIN", JOURNAL OF INVESTIGATIVE DERMATOLOGY, vol. 98, no. 3, 1992, pages 310-314, XP002722925, ISSN: 0022-202X cited in the application

Remarks:
The file contains technical information submitted after the application was filed and not included in this specification

EP 3 027 173 B1

**Description**

This invention relates to a transdermal administration system for use in treatment of diseases of the respiratory tract, particularly chronic bronchopulmonary diseases having a broncho-obstructive component, which is characterised by the use of Doxofylline as the active ingredient.

The invention also relates to Doxofylline in the context of its use in transdermal administration for therapeutic treatment of the abovementioned diseases.

Doxofylline, 2-(7'-theophylline methyl)-1,3-dioxolane is a xanthine derivative. The xanthines are a group of alkaloids commonly used for their effects as mild stimulants and bronchodilators, particularly in treatment of the symptoms of asthma. Numerous drugs, including acefylline piperazine, ambufylline, aminophylline, bamifylline, choline theophyllinate, dyphylline, enprofylline, etamifylline, etophylline, guatilline, proxyphylline, theobromine, theophylline and doxofylline belong to the xanthines family.

Xanthine derivatives are mild stimulants of the central nervous system and also stimulate the respiratory centres. Because of their wide spectrum of effects their therapeutic index is somewhat limited; their serum therapeutic level is generally of the order of 10-20 mg/ml and toxicity symptoms include tremor, nausea, nervousness and tachycardia/arrhythmia.

Various modulated release systems, including transdermal release systems which potentially offer a valuable solution, particularly in terms of compliance on the part of patients and in terms of efficacy of therapeutic response, have also been developed and proposed for xanthines in recent decades.

Despite the considerable interest in the transdermal administration route, the application of this administration route to a wider range of drugs has naturally been limited because of the significant barrier to penetration across the skin, which is primarily associated with the outer corneal layer of the epidermis. In general the daily dose of drug which can be administered by, for example, a transdermal patch is somewhat limited (e.g. 5-10 mg), limiting this method of administration to the most potent drugs.

Since the 90s many scientific studies have examined the percutaneous absorption characteristics of xanthine derivatives in human skin, using theophylline as the reference drug, in an attempt to obtain useful information for the development of transdermal administration systems.

WO2007/066151 A2 discloses the paediatric treatment of respiratory disorders by transdermal administration of therapeutic agents including long-acting beta2-agonists or short-acting beta2-agonists; the therapeutic agent may alternatively be an anti-muscarinic agent, a steroid or may include theophylline or aminophylline.

WO2007/120485 A2 discloses the treatment of pain by co-therapy with an alkyl xanthine bronchodilator which can be doxophylline and an antiepileptic; the administration can be transdermal.

The publication by Ademola J. I. et al. in Journal of Investigative Dermatology (1992), 98(3), 310-14 concludes that a high level of absorption potentiation is required for transdermal theophylline preparations to be able to produce therapeutic concentrations in plasma. The research was therefore aimed at identifying agents potentiating transdermal permeation. The publication by Thakur R. A. in Drug. Dev. Ind. Pharm. 2007, May; 33(5):513-21 examines the in vitro permeation of caffeine, theophylline and theobromine through the human skin with the aid of various chemical potentiating agents, including oleic acid. The administered flow of theobromine and theophylline was nevertheless still insufficient. The subject-matter of the invention resides in a system for transdermal administration comprising doxofylline as the reactive ingredient for the use in the treatment of bronchopulmonary diseases having a broncho-obstructive component. In a further embodiment the invention resides in doxofylline for use in transdermal administration to a patient for the therapeutic treatment of chronic bronchopulmonary diseases, having a broncho-obstructive component. This invention is based on discovery of the fact that in a wholly unexpected way doxofylline has higher transepithelial permeability characteristics, which, in the context of the xanthines make it the ideal active ingredient for the implementation of transdermal administration systems useful in the therapeutic treatment of diseases of the respiratory tract, particularly in adults.

In particular the transdermal administration system which is the subject matter of the invention, containing doxofylline as the active ingredient, brings together the advantages in terms of compliance on the part of the patient and efficacy of therapeutic response which are typical of this form of administration, together with the known therapeutic benefits of doxofylline when administered systemically.

In particular it is known that doxofylline has high bronchodilatory activity with rare collateral effects, even in patients with associated morbidities and receiving multiple therapy. The safety data for the active ingredient and the efficacy of chronic treatment of chronic bronchopulmonary diseases having a broncho-obstructive component render doxofylline an extremely useful drug in long term treatment. In addition to this, long term treatment requires high compliance on the part of the patient, compliance which is well known to be improved through reducing administrations and by time-modulated administration systems. The transdermal administration route for doxofylline in various doses is therefore particularly useful in the long term treatment of nocturnal asthma, chronic pulmonary diseases having a broncho-obstructive component and all forms of broncho-obstructive reaction secondary to bronchial inflammatory diseases, including those secondary to cardiovascular diseases (the so-called chronic pulmonary heart), and in multiple therapy using drugs active on the cardiovascular system

The scope of the invention comprises all transdermal systems for the administration of doxofylline, which make it possible to achieve serum levels of between 0.1 and 300 mg/ml in treated individuals.

The transdermal forms of administration to which the invention relates may be provided in the form of a transdermal patch, for example having two layers (monolithic patch), three layers or in the form of a reservoir.

The formulation of a three-layer transdermal patch comprises:

- a supporting barrier layer (backing) of polymer material, which is flexible and generally non-occlusive, which protects the underlying layer from external agents; this barrier layer may be constructed using polymer materials such as polyurethanes or polyamides or non-woven fabric (artificial silk);
- a second layer of polymer matrix containing doxofylline;
- a third layer of pressure-sensitive adhesive (PSA) containing, for example, polyvinylpyrrolidone or acrylate or polyisobutene, which may be protected by a silicone film, which protects it during storage and is removed prior to use.

The monolithic patch, which currently constitutes the preferred form of implementation, comprises a first backing layer of the type previously described and a second layer which imparts both adhesion and controlled release to the system. The polymer matrix material in which the active ingredient, doxofylline, is dispersed in the monolithic patch form of implementation, or three-layer patch, typically comprises polymer materials such as acrylates, silicones, block copolymers, including in particular polystyrene derivatives; examples of styrene block copolymers having adhesive properties which can be used for monolithic patches comprise, for example, styrene-isoprene-styrene (SIS), styrenebutadiene-styrene (SBS) and styrene-ethylene/butylene-styrene (SEBS) block copolymers.

In a reservoir formulation, which is in itself known, doxofylline is contained in a solution/gel in contact with a membrane which controls its release to the skin.

In one embodiment the high permeability of doxofylline makes it possible to formulate transdermal administration systems which do not include chemical agents potentiating epithelial penetration, that is those agents which increase permeability by fluidising the lipid structure of the corneal layer, such as for example, azone, DMSO, alcohols, fatty acids, terpenes, terpenoids, essential oils and their mixtures. However it is intended that transdermal administration systems comprising one or more potentiating agents such as those mentioned above and their mixtures are included within the scope of the invention.

Tests relating to determination of the permeability of doxofylline in comparison with reference active agents have been carried out using the procedures below.

1. Permeability tests

1.1 Comparison of doxofylline with standard drugs

MDCK cells were kept in Minimal Essential Medium Eagle (EMEM) containing 10% FBS, 2nM of fresh L-glutamine and 2nM of penicillin-streptomycin. The culture inserts were inseminated with 664000 cells per $cm^2$ (0.33 $cm^2$ per insert). The monolayers of MDCK were cultured for three days before use. The monolayers were washed with Hanks Balanced Salt Solution (HBSS +10 nM Hepes pH 7.4) transport medium; the electrical resistance of each monolayer was measured at 37°C using a Millicell ERS-2 (Millipore). Background resistance values were subtracted from the resistance values of treated wells of monolayer. The resulting value was multiplied by the area of the membrane (0.33 $cm^2$) to calculate the transepithelial electrical resistance values (TEER ($\Omega$ $cm^2$)) for each monolayer. TEER values indicate the degree of confluence of the monolayer and the development of tight junctions. If the resistance was < 200 $\Omega$ $cm^2$ the test was continued. MDCK monolayers having TEER values > 200 $\Omega$ $cm^2$ were not used. The donor solutions in the transport test comprised 100 $\mu$M of test compound in the transport medium containing 100 nM of fluorescein isocyanate-dextran. The transport tests were performed using 0.250 ml of apical donor solution and 1 ml of basolateral acceptor solution (transport medium, pH 7.4). The internal standards and doxofylline were tested using five replicates. The monolayers were incubated with donor and acceptor solutions for 120 minutes at 37°C. The basolateral compartments were sampled after 120 minutes and analysed using LC-MS/MS, together with an aliquot of the donor solutions in the transport test (100 $\mu$M of the test compound) corresponding to to (sample at time zero). In the quality check the internal standards (atenolol, dexamethasone, tartrate salt with metoprolol and fluorescein isocyanate-dextran) were tested under the same conditions as the test compound. $P_{app}$ (apparent permeability) values were calculated in accordance with the following equation:

$$P_{A-B} = \frac{\frac{dQ}{dt}}{A \cdot C_A} = \frac{\frac{\Delta Q}{\Delta t}}{A \cdot C_A}$$

in which $dQ/dt$ is the permeation velocity, $C_A$ is the initial concentration in the donor compartment and A is the surface area of the filter. Permeation velocities were calculated by preparing a diagram of the percentage of mass of active ingredient drug (peak area) found in the basolateral compartment as a function of time.

The results for fluorescein isothiocyanate/dextran were used as an internal control for each monolayer, to check the integrity of the tight junction throughout the period of the test. The normal range for fluorescein isocyanate-dextran permeability in MDCK monolayers is approximately 1 to 7 nM/second. The results from MDCK monolayers with fluorescein isothiocyanate/dextran $P_{app}$ < 10 nM/S were not used in calculating the $P_{app}$ for the compound.

LC-MS/MS analyses

The following HPLC conditions were used for all the tests:

- column: Kinetex Cs-2.6 $\mu$M, 2.1 x 50 mm and Phenomenex
- solvent A: 1 mM $NH_4$ AC
- solvent B: 100% $CH_3$ CN.

The gradient used in HPLC separation was:
The To samples (100 $\mu$M) were diluted in 50:50 solvent A: solvent B before analysis.
The dilution factors used were:

- atenolol 1:400
- metoprolol 1:200
- dexamethasone 1:40
- doxofylline 1:26.

The integrated peak area for the LC-MS/MS chromatograms was used as the instrument response (INRE) for relative quantification of the test material.
The permeability of the test material was then evaluated by comparing the INRE for five replicates after two hours incubation, on the basis of the INRE response to the to samples.
The results obtained after incubation with fluorescein isocyanate-dextran demonstrate that the monolayer remained intact (see table 1). The results obtained from incubation with the internal standards and doxofylline show that doxofylline has a high permeability value (table 2).

**Table 1.** Transport well treated with atenolol, metoprolol, dexamethasone and doxofylline for 2 hours; fluorescein isothiocyanate-dextran was read using fluorescence at 520 nM - 490 nM TECAN INFINIT M200 Plate reader to check the integrity of the monolayer.

| | ABS (mean of 5 transport wells) | nanoMolare | $P_{app}$ (nM/sec) |
|---|---|---|---|
| atenolol | 36.4 | 0.10 | 4.2 |
| metoprolol | 34 | 0.10 | 4.2 |
| dexamethasone | 27 | 0.08 | 3.37 |
| doxofylline | 28 | 0.08 | 3.37 |

**Table 2.** $P_{app}$ for atenolol, metoprolol, dexamethasone and doxofylline.

| Compounds | $P_{app}$ (nM/sec) |
|---|---|
| Atenolol (low permeability standard) | 6.82 |
| Metoprolol (high permeability standard) | 65.15 |

(continued)

| Compounds | $P_{app}$ (nM/sec) |
|---|---|
| Dexamethasone (medium permeability standard) | 46.63 |
| Doxofylline | 226.64 |

Through the MTT test using human keratinocyte cell lines (HACAT), human fibroblast cell lines (HFF) and human endothelial cells from the umbilical vein (HUVEC) it was also demonstrated that doxofylline has no cytotoxicity.

1.2 Comparison between doxofylline and theophylline

The skin permeability of doxofylline and theophylline was compared using an *in vitro-ex vivo* experimental protocol across human epidermis using Franz diffusion cells.

The Franz diffusion cells comprise two compartments, one containing the active ingredient (donor vehicle) and the other containing a receiving solution, separated by a slice of human epidermis. The vertical cells used in the actual experimental unit have a wider column than that in the original Franz type of diffusion cell, and the decanter shape was dispensed with. These have a diffusion area of 0.636 cm$^2$ and a receiving compartment of approximately 3.0 ml. The receiving volume in each cell is individually calibrated and equipped with a magnetic stirrer.

Materials:

Theophylline WS RSNC 004 A, batch No. TB-120076
Doxofylline WS, batch No. 15530613

In vitro penetration test

Human epidermis was selected as the membrane for skin permeation studies. The skin used in the studies was obtained from the abdominal skin of a donor who underwent cosmetic surgery. The skin samples were prepared following an internal standard procedure. The full thickness of the skin was sealed in evacuated plastic bags and frozen at -20°C within 6 hours of removal. Before the experiments the skin was thawed out at ambient temperature and excess fat was completely removed. The sections of skin were cut into squares of approximately 2.5 cm$^2$ and after the skin had been immersed in water at 60°C for one minute the epidermis was separated off from the remaining tissue using forceps. The integrity of the skin was evaluated by measuring the impedance of each sample (Agilent LCR Meter 4263B).

Subsequently the epidermis was mounted in the Franz diffusion cell, the receiving compartment of which was filled with saline solution (NaCl 0.9% w/v) filtered using a 0.22 $\mu$m filter (VWR, nylon membrane). Particular attention was paid to avoiding air bubbles between the cover and the epidermis in the receiving compartment. The top and bottom parts of the Franz cell were sealed with Teflon and Parafilm® (Pechiney Plastic Packaging Company, Chicago, USA) and attached together by means of a clamp with the epidermis acting as a seal between the donor and receiving compartments. The donor compartment was then filled with 0.5 ml of a solution of theophylline in water or in triacetin (4% w/v). A precipitate was observed in these solutions, and these were therefore filtered using a 0.45 $\mu$m filter (VWR, polypropylene membrane) before being placed in the cells. A portion of each was preserved and diluted 1:2 in water or triacetin.

The system was maintained at 37°C using a circulating water bath, so that the temperature of the surface of the epidermis was 32 $\pm$ 1°C during the test. At predetermined times (1, 3, 6, 24 hours) samples of 0.2 ml were taken from the receiving compartment and replaced with fresh receiving medium. "Sink" conditions were maintained during the tests.

The experimental conditions are summarised in the table below:

| Method | Modified Franz diffusion systems |
|---|---|
| Volume of the receiving compartment | ≈ 3 ml |
| Dimensions of the test sample | 0.636 cm$^2$ |
| Medium | Saline solution |

(continued)

| Method | Modified Franz diffusion systems |
|---|---|
| Temperature | $32\pm1°C$ |
| Volume of sample removed | 0.2 ml |
| Sampling times | 1h, 3h, 6h, 24h |

The tests performed using theophylline were repeated in the same way using a solution of doxofylline in water or triacetin (4% w/v).

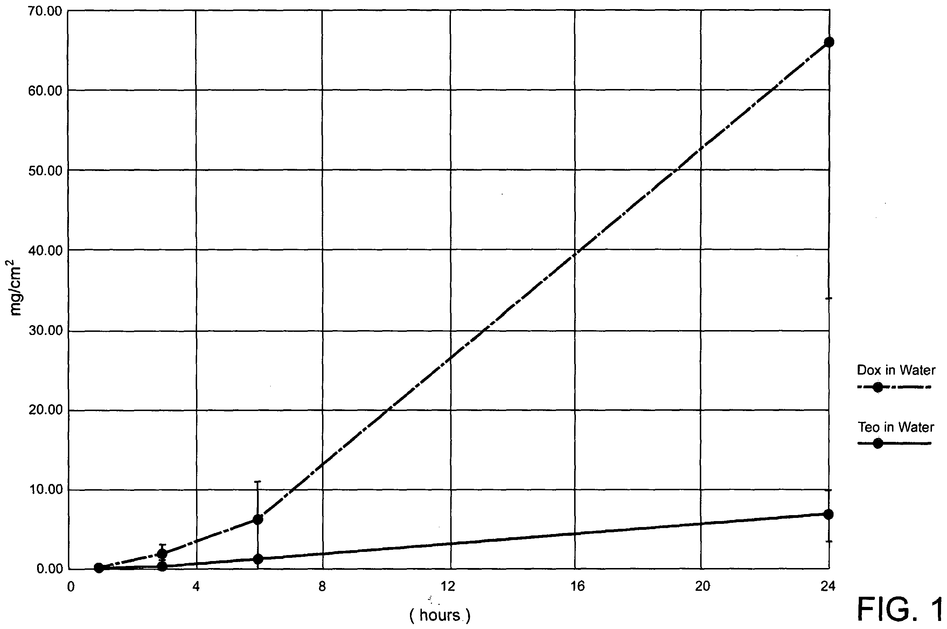

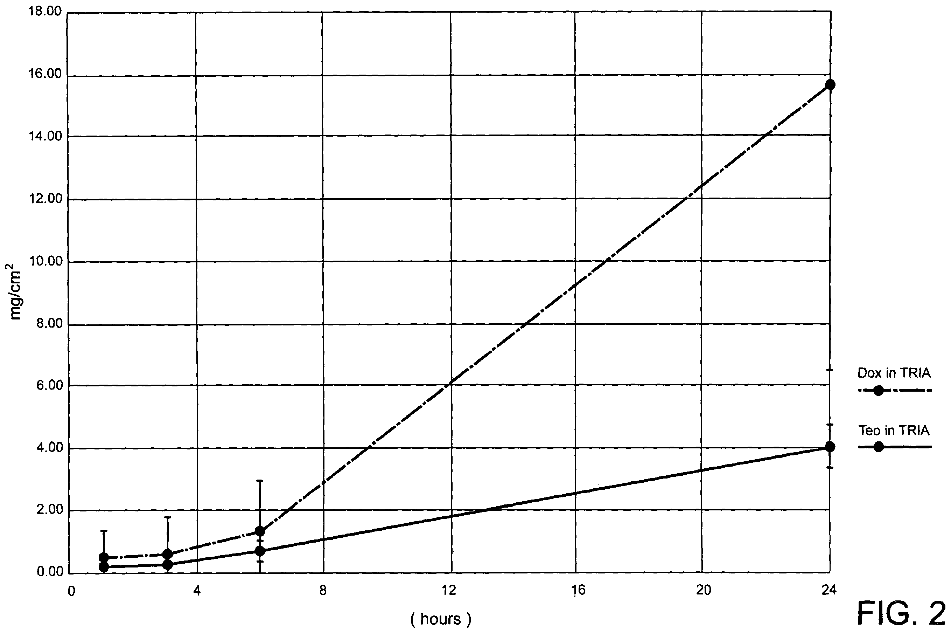

The results obtained are shown in the graphs in Figures 1 and 2.

The graph in Figure 1 shows the values of doxofylline and theophylline permeate expressed as micrograms/cm$^2$ of surface area over time (4 sample times up to 24 hours) with aqueous solvent.

The graph in Figure 2 similarly shows the values for doxofylline and theophylline permeate in triacetin solvent.

The results clearly demonstrate that the skin permeability values for doxofylline are substantially higher than those for theophylline in both the solvents used.

## Claims

1. System for transdermal administration comprising doxofylline as the active ingredient for the use in the treatment of bronchopulmonary diseases having a broncho-obstructive component.

2. System for administration according to claim 1, for use in the long term treatment of asthma, nocturnal asthma, chronic lung diseases having a broncho-obstructive component, forms of broncho-obstructive reaction secondary to bronchial inflammatory diseases or cardiovascular diseases, optionally in multiple treatment with drugs active on the cardiovascular system.

3. System for administration according to claim 1 or 2, in the form of a reservoir.

4. System for administration according to claim 1 or 2, in the form of a transdermal patch comprising a first supporting polymer barrier layer, a second layer of polymer matrix containing doxofylline and a third layer comprising a pressure-sensitive adhesive.

5. System for administration according to claim 1 or 2, in the form of a transdermal patch comprising a supporting layer of polymer material and a second polymer layer of matrix including doxofylline, having adhesive properties.

6. System for administration according to claim 4 or 5, in which the matrix polymer is selected from the group comprising acrylic and silicone (co)polymers and styrene block copolymers.

7. System for administration according to any one of the preceding claims, without any agents potentiating permeation of the active ingredient.

8. Doxofylline for use in transdermal administration to a patient for the therapeutic treatment of chronic bronchopulmonary diseases, having a broncho-obstructive component.

9. Doxofylline according to claim 8, for use in the therapeutic treatment of asthma, nocturnal asthma, forms of broncho-obstructive reaction secondary to bronchial inflammatory diseases and secondary to cardiovascular diseases.

## Patentansprüche

1. System zur transdermalen Verabreichung, das Doxofyllin als Wirkstoff umfasst, zur Verwendung bei der Behandlung von bronchopulmonalen Erkrankungen mit einer bronchoobstruktiven Komponente.

2. System zur Verabreichung nach Anspruch 1 zur Verwendung bei der Langzeitbehandlung von Asthma, nächtlichem Asthma, chronischen Lungenerkrankungen mit einer bronchoobstruktiven Komponente, Formen einer bronchoobstruktiven Reaktion sekundär zu bronchialen Entzündungserkrankungen oder kardiovaskulären Erkrankungen, optional in einer Mehrfachbehandlung mit Arzneistoffen, die bezüglich des kardiovaskulären Systems wirksam sind.

3. System zur Verabreichung nach Anspruch 1 oder 2 in der Form eines Reservoirs.

4. System zur Verabreichung nach Anspruch 1 oder 2 in der Form eines Transdermalpflasters, das eine erste stützende Polymerbarriereschicht, eine zweite Schicht aus einer Polymermatrix, die Doxofyllin enthält, und eine dritte Schicht umfasst, die ein druckempfindliches Haftmittel umfasst.

5. System zur Verabreichung nach Anspruch 1 oder 2 in der Form eines Transdermalpflasters, das eine stützende Schicht aus einem Polymermaterial und eine zweite Polymerschicht aus einer Matrix umfasst, die Doxofyllin umfasst, und Hafteigenschaften aufweist.

6. System zur Verabreichung nach Anspruch 4 oder 5, bei dem das Matrixpolymer aus der Gruppe, bestehend aus Acryl- und Silikon(co)polymeren und Styrolblockcopolymeren, ausgewählt ist.

7. System zur Verabreichung nach einem der vorhergehenden Ansprüche ohne irgendwelche Mittel, welche die Permeation des Wirkstoffs verstärken.

8. Doxofyllin zur Verwendung bei der transdermalen Verabreichung an einen Patienten zur therapeutischen Behandlung von chronischen bronchopulmonalen Erkrankungen mit einer bronchoobstruktiven Komponente.

9. Doxofyllin nach Anspruch 8 zur Verwendung bei der therapeutischen Behandlung von Asthma, nächtlichem Asthma, Formen einer bronchoobstruktiven Reaktion sekundär zu bronchialen Entzündungserkrankungen und sekundär zu kardiovaskulären Erkrankungen.


**Revendications**

1. Système d'administration transdermique comprenant de la doxofylline en tant que principe actif pour son utilisation dans le traitement de maladies broncho-pulmonaires avec obstruction bronchique.

2. Système d'administration selon la revendication 1, pour son utilisation dans le traitement à long terme de l'asthme, de l'asthme nocturne, de maladies pulmonaires chroniques avec obstruction bronchique, de formes de réaction broncho-obstructive secondaire à des maladies bronchiques inflammatoires ou des maladies cardiovasculaires, facultativement en traitement multiple avec des médicaments actifs sur le système cardiovasculaire.

3. Système d'administration selon la revendication 1 ou 2, sous la forme d'un réservoir.

4. Système d'administration selon la revendication 1 ou 2, sous la forme d'un patch transdermique comprenant une première couche polymère de support formant barrière, une deuxième couche de matrice polymère contenant de la doxofylline et une troisième couche comprenant un adhésif autocollant.

5. Système d'administration selon la revendication 1 ou 2, sous la forme d'un patch transdermique comprenant une couche de support de matériau polymère et une deuxième couche polymère de matrice comprenant de la doxofylline, ayant des propriétés adhésives.

6. Système d'administration selon la revendication 4 ou 5, dans lequel la matrice polymère est sélectionnée dans le groupe comprenant des (co)polymères d'acrylique et de silicone et des copolymères séquencés de styrène.

7. Système d'administration selon l'une quelconque des revendications précédentes, sans agent de potentialisation de la perméation du principe actif.

8. Doxofylline pour son utilisation dans l'administration transdermique à un patient pour le traitement thérapeutique de maladies broncho-pulmonaires chroniques avec obstruction bronchique.

9. Doxofylline selon la revendication 8, pour son utilisation dans le traitement thérapeutique de l'asthme, de l'asthme nocturne, de formes de réaction broncho-obstructives secondaires à des maladies bronchiques inflammatoires et secondaires à des maladies cardiovasculaires.

FIG. 1

EP 3 027 173 B1

FIG. 2

EP 3 027 173 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007066151 A2

- WO 2007120485 A2

**Non-patent literature cited in the description**

- **ADEMOLA J. I. et al.** *Journal of Investigative Dermatology,* 1992, vol. 98 (3), 310-14

- **THAKUR R. A.** *Drug. Dev. Ind. Pharm.,* May 2007, vol. 33 (5), 513-21